(19) Europäisches Patentamt
European Patent Office
Office européen des brevets

(11) Numéro de publication: **0 388 267**
**A1**

(12) **DEMANDE DE BREVET EUROPEEN**

(21) Numéro de dépôt: **90400639.2**

(51) Int. Cl.5: **C12P 1/00**

(22) Date de dépôt: **12.03.90**

(30) Priorité: **13.03.89 FR 8903228**

(43) Date de publication de la demande:
**19.09.90 Bulletin 90/38**

(84) Etats contractants désignés:
**AT BE CH DE ES FR GB IT LI NL SE**

(71) Demandeur: **SOCIETE NATIONALE ELF AQUITAINE**
**Tour Elf 2 Place de la Coupole La Défense 6**
**F-92400 Courbevoie(FR)**

(72) Inventeur: **Bergel, Alain**
**42, rue Pigni**
**F-31500 Toulouse(FR)**
Inventeur: **Comtat, Maurice**
**Résidence du Midi, Impasse Delfour**
**F-31400 Toulouse(FR)**
Inventeur: **Seris, Jean-Louis**
**Lot, Parenche No. 15, Chemin Vignats**
**F-64110 Jurancon(FR)**

(74) Mandataire: **Ohresser, François**
**Société Nationale Elf Aquitaine Division**
**Proprieté Industrielle Tour Elf**
**F-92078 Paris La Défense Cédex 45(FR)**

(54) **Procédé de régénération par réduction électrochimique d'un cofacteur pyridinique.**

(57) L'invention a pour objet un procédé de régénération électrochimique de cofacteurs pyridiniques.
Le procédé de l'invention est caractérisé par la mise en oeuvre d'une catalyse par double médiation au moyen d'une flavine et d'un enzyme oxydo-réductase.

EP 0 388 267 A1

# PROCEDE DE REGENERATION PAR REDUCTION ELECTROCHIMIQUE D'UN COFACTEUR PYRIDINIQUE

La présente invention a pour objet un procédé de régénération et de réduction par voie électrochimique du nicotinamide adénine dinucléotide, cofacteur pyridinique.

L'utilisation des enzymes d'oxydo-réduction en synthèse organique se développe rapidement. Ces procédés de synthèse enzymatique mettent en oeuvre des cofacteurs pyridiniques tels que le NADH (nicotinamide adénine dinucléotide) ou le NADPH (nicotinamide adénine dinucléotide phosphate) impliqués dans le mécanisme d'oxydo-réduction. Toutefois, la transposition des résultats obtenus en laboratoire à une plus grande échelle nécessite, d'une part la régénération des cofacteurs pyridiniques beaucoup trop chers pour être utilisés en quantités stoechiométriques et, d'autre part des moyens fiables de contrôle des réactions.

C'est la raison pour laquelle on assiste au développement de nombreux travaux pour la recherche des conditions optimales de régénération de ces cofacteurs. Celle-ci peut être réalisée par voie chimique, enzymatique ou électrochimique. L'analyse des avantages et inconvénients de ces diverses voies montre qu'aucune technique générale de régénération ne donne entière satisfaction et il convient dans chaque cas particulier d'envisager l'optimisation de l'ensemble réaction directe/régénération.

Pour la réduction de $NAD^+$, les méthodes enzymatiques ont jusqu'ici donné les meilleurs résultats. Parmi les systèmes utilisés, on peut citer les couples substrats-enzymes suivants : formiate-formiate déshydrogénase, glucose 6.phosphate-glucose-6.phosphate déshydrogénase, glucose-glucose déshydrogénase, éthanol-alcool déshydrogénase, hydrogène-hydrogénase.

Les procédés électrochimiques semblent attractifs, au moins théoriquement, parce qu'ils permettent de fixer très facilement la vitesse de régénération par le choix du potentiel d'électrode. Par ailleurs, ils offrent la possibilité d'un contrôle facile de la réaction par la mesure de l'intensité d'électrolyse au cours du temps. Toutefois, les avantages sont limités par l'incompatibilité de certains réactifs susceptibles de réagir directement sur l'électrode portée au potentiel de réduction ; la pollution de l'électrode par les produits et les réactifs adsorbables et le manque de sélectivité sont d'autres inconvénients majeurs. Cette dernière difficulté est particulièrement sensible pour la réduction en raison de la formation par transfert monoélectronique de l'intermédiaire radicalaire $NAD^\bullet$ susceptible de se dimériser rapidement. Le radical apparaît sur les électrodes quelle que soit leur nature.

La maîtrise du transfert électronique entre les électrodes et $NAD^+$ est importante pour la mise en oeuvre du processus de régénération de NADH par réduction de $NAD^+$.

Pour pallier ces inconvénients et améliorer le transfert, il a été proposé d'utiliser des médiateurs tels que le méthylviologène, des complexes du rhodium ou l'aminoptéridine. Il faut observer cependant que le transfert avec médiateur n'améliore que très modestement les performances, conduit à la passivation des électrodes et à l'apparition de produits secondaires. On peut citer à titre d'exemple les travaux de S.KWEE et H.LUND (Bioélectrochem. Bioeng. 1974,1,87-95). D'autres auteurs ont proposé d'utiliser pour la réaction de réduction un couple méthylviologène-enzyme : H.SIMON, H.GUNTER, J.BADER et W.TISCHER (Angew. Chem. 1981, 20, 961-63) qui décrivent la catalyse de la réaction : $NAD^+ + H^+ + 2MV^{\bullet +}$ ---> NADH + $2MV^{2+}$ par une énoate réductase (Clostridia) et la régénération de $MV^{2+}$ sur une cathode :

$MV^{2+} + e$ ---> $MV^{\bullet +}$

Les problèmes posés par les réactions secondaires dues à $MV^{\bullet +}$ limitent l'utilisation de ce type de processus.

La demanderesse a, pour sa part, proposé dans une demande de brevet français en cours n° 88 11933 du 13.09.1988 un procédé de régénération de NADH par réduction électrochimique de $NAD^+$ utilisant une enzyme hydrogénase cytoplasmique agissant comme médiateur en assurant un relais électronique entre l'électrode et le cation $NAD^+$ qui évite ainsi la formation du dimère $(NAD)_2$.

La présente invention a pour but de pallier les inconvénients précédents et propose un système catalytique à double médiation permettant la régénération du cofacteur par réduction de $NAD^+$, en évitant la formation des produits secondaires, mais qui, par ailleurs, peut être utilisé dans un processus d'oxydation de NADH.

L'invention a pour objet un procédé de régénération par réduction du nicotinamide adénine dinucléotide ($NAD^+$) ou ses dérivés tels que le nicotinamide adénine dinucléotide phosphate ($NADP^+$) par mise en oeuvre de la réaction : $NAD(P)^+ + H^+ + 2e$ ---> NAD(P)H caractérisé par l'utilisation dans le milieu réactionnel de l'association d'une flavine et d'une enzyme oxydo-réductase (EOR).

La catalyse des réactions se fait par transfert d'électrons entre le métal de l'électrode, la flavine et le couple $NAD^+$/NADH. Le mécanisme mis en jeu peut être sommairement représenté sur le schéma suivant dans lequel la flavine est symbolisée par Fla :

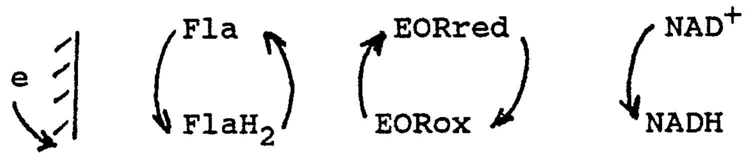


Lorsqu'on réalise la réduction de $NAD^+$ par $FlaH_2$, il est nécessaire de disposer dans le milieu d'une solution à forte concentration de $FlaH_2$. Le processus suivant est alors mis en oeuvre :

$$Fla + 2H^+ + 2e \text{ ----------> } FlaH_2$$

EOR

$$FlaH_2 + NAD^+ \text{ ------------> } Fla + NADH + H^+ \qquad (1)$$

L'avantage essentiel réside dans l'absence du dimère $(NAD)_2$ qui apparaît habituellement lors de la réduction directe sur une électrode non modifiée. La surtension en réduction est diminuée de 0,1 volt par rapport au processus de réduction directe.

Le procédé de l'invention est mis en oeuvre en présence d'une flavine, c'est-à-dire d'un composé de la famille des riboflavines telle que décrite dans le Kirk-Othmer 3ème édition vol. 24 pp 108-121, susceptible de passer aisément par transfert électronique ou catalyse enzymatique d'une forme oxydée Fla à une forme réduite $FlaH_2$ et réciproquement. On utilise de préférence des composés tels la flavine mononucléotide (FMN) ou la flavine adénine dinucléotide (FAD).

Ces molécules biologiques, qui sont généralement mises en oeuvre dans les organismes où interviennent les enzymes utilisées, sont naturellement plus biocompatibles que les composés organiques, tels que le méthylviologène, et présentent donc l'avantage de minimiser les risques de réactions secondaires.

Le procédé de l'invention utilise dans le milieu, conjointement avec la flavine, une enzyme, désignée conventionnellement par EOR, susceptible de catalyser la réaction d'oxydo-réduction permettant de transformer $NAD^+$ et $FlaH_2$ en NADH et Fla, c'est-à-dire simultanément de réduire $NAD^+$ et d'oxyder $FlaH_2$.

Parmi les EOR utilisables dans le procédé de l'invention, on peut citer la FMN-oxydoréductase de Photobactérium pischiri, la ferrédoxine-$NADP^+$- réductase (FNR) extraite de feuilles d'épinards et la formiate deshydrogénase (FDH) de Pseudomonas oxalaticus. Cette dernière enzyme catalyse l'oxydation de l'ion formiate selon la réaction suivante :

$$HCO_2^- + NAD^+ \xrightarrow{FDH} CO_2 + NADH.$$

Selon la littérature, cette enzyme est très spécifique de l'ion formiate et seuls l'ion formiate et NADH sont susceptibles de jouer le rôle de donneur d'électron alors qu'elle admet un nombre d'accepteurs d'électron beaucoup plus important (flavines, colorants, oxygène).

C'est aussi avec surprise qu'il a été constaté que cette enzyme était capable de catalyser une réaction de réduction dans laquelle n'intervient pas l'ion formiate.

On utilisera de préférence l'enzyme formiate deshydrogénase extraite à partir de Pseudomonas oxalaticus. Cette enzyme a un poids moléculaire Mw de 315.000 Daltons et comporte deux flavines mononucléotides, de 18 à 25 atomes de fer et de 15 à 20 atomes de soufre, organisés en deux fois deux sous unités de Mw 100.000 et 59.000 Daltons.

Il est essentiel que les deux éléments, flavine et EOR, soient mis en oeuvre simultanément ; en effet, le processus électrochimique ne permet pas d'obtenir une réduction convenable de $NAD^+$ en l'absence, soit de la flavine, soit de l'enzyme EOR.

L'une des caractéristiques importantes du procédé de l'invention est la réalisation de la réaction de réduction de $NAD^+$ au moyen d'un système catalytique généralement utilisé pour l'oxydation de NADH, par inversion de l'équilibre grâce à l'utilisation de l'électrochimie en couche mince. La réaction d'oxydo-réduction (1) décrite page 3 est une réaction équilibrée dont l'équilibre thermodynamique favorise la réaction d'oxydation de NADH en $NAD^+$. Lorsqu'on cherche à la mettre en oeuvre dans le sens de la

réduction il est important de disposer dans le milieu d'une forte concentration en $FlaH_2$.

L'électrochimie en couche mince est caractérisée par la mise en oeuvre d'une cellule telle que la surface (S) de l'électrode est grande en regard du volume réactionnel (V). On estime que le rapport S/V d'une telle cellule est compris entre 1.000 et 10.000 $m^{-1}$.

Ce dispositif permet de conserver une concentration de Fla pratiquement nulle dans l'ensemble du volume réactionnel et, par incidence, assure une concentration de $FlaH_2$ très élevée et homogène qui provoque le déplacement de l'équilibre :

$FlaH_2 + NAD^+$ ----------> $Fla + NADH + H^+$

Le procédé de l'invention sera réalisé à un pH sensiblement neutre. Il sera en général compris entre 5 et 9 correspondant à la stabilité des enzymes utilisées dans le processus et à leur pH optimal de fonctionnement.

Les quantités respectives de $NAD^+$ et de FAD dans le milieu réactionnel seront choisies de manière à optimiser la vitesse globale de la réaction et ceci en fonction des cinétiques des deux réactions successives en présence de l'enzyme EOR considérée.

Lorsque, dans le cadre d'un procédé de réduction enzymatique d'un substrat on est amené à régénérer NADH par réduction de $NAD^+$ en utilisant le procédé de l'invention, les quantités de flavine et de $NAD^+$ utilisées dans le milieu réactionnel sont faibles comparées aux quantités de substrat à transformer. En effet, la flavine et $NAD^+$ sont constamment transformées et régénérées et jouent simplement le rôle de catalyseur dans la transformation, l'agent réducteur étant la cathode qui fournit les électrons nécessaires.

Le rapport pondéral substrat/flavine ou substrat/ $NAD^+$ sera en général compris entre 100 et 10.000.

En outre, l'enzyme EOR sera généralement utilisée en quantité catalytique, le milieu en contiendra en général de 10 à 500 U/ml.

Les caractéristiques du procédé de l'invention seront mieux comprises à la lecture des exemples ci-après, donnés à titre simplement illustratif.

Exemples 1 à 11

Cette série d'exemples illustre la réduction par voie électrochimique de $NAD^+$ par double médiation au moyen de $FADH_2$ et de l'enzyme formiate deshydrogénase.

Les conditions opératoires générales de ces exemples sont les suivantes :

- électrode au platine mise en oeuvre en couche mince : rapport V/S de l'ordre de 3 500 $m^{-1}$
- concentration en $NAD^+$ : 5mM
- milieu : tampon phosphate 0,2M, pH : 7,0

Le milieu contient également :

- l'enzyme formiate deshydrogénase FDH,
- et une flavine : FAD en quantité variable selon les exemples ainsi que cela est précisé dans le tableau 1 ci-dessous.

En pratique, on forme tout d'abord $FADH_2$ par réduction électrochimique de FAD, $FADH_2$ réduisant ensuite $NAD^+$ en NADH sous l'action de l'enzyme FDH.

La transformation par réduction est évaluée par la vitesse initiale de formation de NADH(v) qui est mesurée en suivant l'évolution de l'absorbance à 340 nm pendant une électrolyse effectuée à - 0,6V/ECS (Electrode au Calomel saturée).

Les résultats obtenus en faisant varier d'une part la concentration en flavine FAD et d'autre-part la concentration en FDH sont regroupés dans le tableau 1 ci-dessous

Tableau 1

| Réduction de $NAD^+$ par double médiation $FADH_2$ + FDH | | | | | | | | | | | |
|---|---|---|---|---|---|---|---|---|---|---|---|
| FAD (mM) | 0 | 0 | 3,0 | 7,0 | 1,75 | 2,25 | 3,0 | 4,5 | 5,25 | 6,0 | 8,0 |
| FDH (mg/ml) | 40 | 60 | 0 | 0 | 40 | 40 | 40 | 40 | 40 | 40 | 40 |
| v ($\mu$M. $min^{-1}$) | 0 | 0 | 0 | 0 | 5,3 | 6,6 | 7,1 | 10,0 | 12,4 | 13,8 | 15,4 |

Les exemples 1 à 4 comparatifs montrent qu'en l'absence soit de la flavine, soit de l'enzyme, il ne se forme pas de NADH. La présence simultanée des deux molécules est indispensable à la mise en oeuvre du

procédé.

Les exemples 5 à 11 illustrent l'influence de la concentration en FAD sur la formation de NADH.

Exemple 12

Cet exemple illustre la régénération par réduction d'un cofacteur pyridinique par voie électrochimique en couche mince mettant en oeuvre comme flavine la flavine mononucléotide (FMN) et l'enzyme NADH : FMN oxydo-réductase de Photobactérium pischeri.

Les conditions opératoires étaient les suivantes :

$NAD^+$ : 5mM, FMN : 2mM, Enzyme : 1,3 U/ml,
Milieu tampon phosphate 0,1M à pH 7,2
Electrolyse à - 0,65 V/ECS
Vitesse initiale de réduction de $NAD^+$ : $2{,}6.10^{-3}$ $\mu$ mole.$mn^{-1}$.$U^{-1}$

Exemple 13

Cet exemple illustre la régénération de NADPH (Nicotinamide adénine dinucléotide phosphate) par réduction de $NADP^+$ par voie électrochimique en couche mince mettant en oeuvre FMN comme flavine et l'enzyme visée à l'exemple 12 ci-dessus.

Les conditions opératoires étaient les suivantes :

$NADP^+$ : 5mM, FMN : 2,5mM, Enzyme : 2,6 U/ml,
Milieu : tampon phosphate 0,1M, pH : 7,2
Electrolyse à - 0,65 V/ECS
La vitesse initiale de réduction de $NADP^+$ : 1,2 $10^{-3}$ $\mu$ mole $mn^{-1}$ $U^{-1}$.

Exemple 14

Cet exemple illustre la régénération de NADPH par réduction de $NADP^+$ par voie électrochimique en couche mince en mettant en oeuvre FMN comme flavine et l'enzyme ferrédoxine-$NADP^+$-réductase extraite de feuilles d'épinards.

Les conditions opératoires sont les suivantes :

$NADP^+$ : 5 mM ; FMN : 2,5 mM ; enzyme : 4 U/ml
Milieu : tampon phosphate 0,1 M, pH 7,2
Electrolyse à -0,65 V/ECS
La vitesse initiale de réduction de $NADP^+$ : 5,5 $10^{-3}$ $\mu$ mole.$min^{-1}$.$U^{-1}$.

## Revendications

1 - Procédé de régénération par réduction électrochimique du cofacteur pyridinique Nicotinamide Adénine Dinucléotide ou ses dérivés caractérisé en ce qu'il est réalisé dans un milieu comprenant une flavine et une enzyme oxydo-réductase.

2 - Procédé selon la revendication 1 caractérisé en ce que la flavine est la flavine adénine dinucléotide (FAD).

3 - Procédé selon la revendication 1 caractérisé en ce que la flavine est la flavine mononucléotide (FMN).

4 - Procédé selon l'une des revendications 1 à 3 caractérisé en ce que l'enzyme oxydo-réductase est une formiate deshydrogénase (FDH) extraite de Pseudomonas Oxalaticus.

5 - Procédé selon l'une des revendications 1 à 3 caractérisé en ce que l'enzyme oxydo-réductase est une NAD-FMN oxydo-réductase extraite de Photobactérium pischeri.

6 - Procédé selon l'une des revendications 1 à 3 caractérisé en ce que l'enzyme oxydo-réductase est la ferrédoxine-$NADP^+$-réductase (FNR).

7 - Procédé selon l'une des revendications 1 à 6 caractérisé en ce qu'il est mis en oeuvre dans un dispositif électrochimique à couche mince.

8 - Procédé selon la revendication 7 caractérisé en ce que le dispositif est pourvu d'une cellule et d'une

électrode au platine telle que le rapport S/V est compris entre 1.000 et 10.000 $m^{-1}$.

9 - Procédé selon l'une des revendications 1 à 6 caractérisé en ce que la teneur du milieu en enzyme oxydo-réductase est comprise entre 10 et 500 U/ml.

Office européen des brevets

# RAPPORT DE RECHERCHE EUROPEENNE

Numero de la demande

EP 90 40 0639

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 102, no. 22, 3 juin 1985, page 170, résumé no. 187946c, Columbus, Ohio, US; C. LAANE et al.: "In vitro synthesis of (bio)chemicals using flavincontaining enzymes", & FLAVINS FLAVOPROTEINS, PROC. INT. SYMP., 8th 1984, 879-92 | 1 | C 12 P 1/00 |
| A | CHEMICAL ABSTRACTS, vol. 85, no. 15, 11 octobre 1987, page 245, résumé no. 106033h, Columbus, Ohio, US; M.O. MANSSON et al.: "Continuous regeneration of NAD(P)+ by flavines covalently bound to sepharose", & BIOTECHNOL. BIOENG. 1976, 18(8), 1145-59 | 1 | |
| A | CHEMICAL ABSTRACTS, vol. 85, no. 25, 20 décembre 1976, résumé no. 188336w, Columbus, Ohio, US; J.B. JONES et al.: "Nicotinamide coenzyme regeneration. Flavin mononucleotide (riboflavin phosphate) as an efficient, economical, and enzyme-compatible recycling agent", & CAN. J. CHEM. 1976, 54(19), 2969-73 | 1 | **DOMAINES TECHNIQUES RECHERCHES (Int. Cl.5)**<br>C 12 P |
| A | APPLIED BIOCHEMISTRY AND BIOTECHNOLOGY, vol. 14, 1987, pages 147-152,179-180, The Humana Press Inc.; H.K. CHENAULT et al.: "Regeneration of nicotinamide cofactors for use in organic synthesis" * Pages 147-150,179-180 * | 1 | |

Le présent rapport a été établi pour toutes les revendications

| Lieu de la recherche | Date d'achèvement de la recherche | Examinateur |
|---|---|---|
| LA HAYE | 22-06-1990 | DELANGHE L.L.M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)